# EUROPÄISCHE PATENTSCHRIFT

(11) **EP 4 252 810 B1**
(45) Veröffentlichungstag und Bekanntmachung des Hinweises auf die Patenterteilung: **31.12.2025**
(21) Anmeldenummer: 23163184.7
(22) Anmeldetag: 21.03.2023
(51) Int. Cl.: A61M 16/08

(54) **BAUGRUPPE FÜR EIN PATIENTEN-BEATMUNGSSYSTEM SOWIE TEMPERATURSENSOREINRICHTUNG ZUM EINSTECKEN IN EINE SENSORAUFNAHME EINES KONNEKTORS EINER DERARTIGEN BAUGRUPPE**
ASSEMBLY FOR A PATIENT VENTILATION SYSTEM AND TEMPERATURE SENSOR DEVICE FOR PLUGGING INTO A SENSOR RECEPTACLE OF A CONNECTOR OF SUCH AN ASSEMBLY
ENSEMBLE POUR UN SYSTÈME RESPIRATOIRE DE PATIENT AINSI QUE DISPOSITIF DE CAPTEUR DE TEMPÉRATURE DESTINÉ À ÊTRE INSÉRÉ DANS UN LOGEMENT DE CAPTEUR D'UN CONNECTEUR D'UN TEL ENSEMBLE

(30) Priorität: 28.03.2022 DE 102022203010
(43) Veröffentlichungstag der Anmeldung: 04.10.2023
(73) Patentinhaber: New Ventures GmbH, 95111 Rehau (DE); Raumedic AG, 95213 Münchberg (DE)
(72) Erfinder: Ehrenpfordt, Ricardo, 08056 Zwickau (DE); Ranfeld, Constanze, 95111 Rehau (DE); Golbs, Lydia, 95182 Döhlau (DE); Ruhland, Thomas, 95444 Bayreuth (DE)
(74) Vertreter: Schmitt-Nilson Schraud Waibel Wohlfrom Patentanwälte Partnerschaft mbB

(56) Entgegenhaltungen:
- WO-A1-2022/069428
- CN-A- 113 117 197
- CN-U- 213 852 621
- DE-U1- 202005 008 156
- US-A1- 2017 197 055
- US-A1- 2020 023 156
- US-A1- 2021 108 640

## Beschreibung

Die Erfindung betrifft eine Baugruppe für ein Patienten-Beatmungssystem. Ferner betrifft die Erfindung eine Temperatursensoreinrichtung zum Einstecken in eine Sensoraufnahme eines Konnektors einer derartigen Baugruppe.

Komponenten für ein Patienten-Beatmungssystem sind bekannt aus der CN 213 852 621 U, aus der WO 2007/051 230 Al, aus der DE 10 2019 216 489 Al, aus der US 9,903,371 B2 und aus der US 2019/0 290 866 A1. Vom Markt her ist weiterhin ein Patienten-Beatmungssystem "VentStar Helix" bekannt.

Aus der CN 213 852 621 U ist ein Ventilatorschlauch für eine Beatmungsmaschine bekannt. Aus der US 2021/108640 A1 ist ein Konnektor für Luftzufuhrleitungen in Beatmungsgeräten bekannt. Aus der DE 20 2005 008156 U1 ist ein beheizbarer Schlauch für die Beatmung oder Atemtherapie bekannt. Aus der CN 113 117 197 A ist ein Konnektor für ein Beatmungsgerät bekannt.

Die US 2017/197055 A1 zeigt eine Baugruppe für ein Patienten-Beatmungssystem mit den Merkmalen des Oberbegriffs von Anspruch 1.

Es ist eine Aufgabe der vorliegenden Erfindung, eine Baugruppe für ein Patienten-Beatmungssystem zu schaffen, über die eine Anzahl von zusammenzusteckenden, luftführenden Komponenten des Patienten-Beatmungssystems verringert ist.

Diese Aufgabe ist erfindungsgemäß gelöst durch eine Baugruppe mit den im Anspruch 1 angegebenen Merkmalen.

Erfindungsgemäß wurde erkannt, dass es möglich ist, innerhalb einer Baugruppe für ein Patienten-Beatmungssystem mit einem integralen Konnektor, der dazu dient, einen Atemluft-Schlauchabschnitt mit einem Patienten-Interface zu verbinden, diesen Konnektor gleichzeitig mit einer Sensoraufnahme für einen Temperatursensor auszustatten. Wahlweise kann der Temperatursensor dann, soweit eine Temperaturmessung erforderlich ist, in die Sensoraufnahme eingesteckt sein oder die Sensoraufnahme kann mit einem Blind-Deckel verschlossen sein, soweit keine Temperaturmessung notwendig ist. Mit einer derartigen Baugruppe wird ein separater Sensor-Konnektor vermieden, über den die Baugruppe dann mit dem Patienten-Interface verbunden wäre. Die Sensoraufnahme kann einstückig in ein Gehäuse des Konnektors eingeformt sein. Die Sensoraufnahme kann beispielsweise Ergebnis eines Spritzguss-Herstellungsverfahrens des Gehäuses des Konnektors sein.

Die Luftführungs-Konnektorkomponente des Konnektors einerseits und der Atemluft-Schlauchabschnitt andererseits sind einstückig miteinander verbunden.

Bei der weiteren, die Beatmungsluft führenden Komponente des Patienten-Beatmungssystems, zu der der Konnektor der Baugruppe eine Verbindung herstellt, kann es sich um ein Patienten-Interface handeln. Alternativ kann es sich bei dieser weiteren Komponente z. B. um einen Befeuchter handeln.

Kontaktpins nach Anspruch 1 ermöglichen eine Kommunikation der Temperatursensoreinrichtung mit einer zentralen Steuerung des Patienten-Beatmungssystems insbesondere über Mantel-Drähte, die im Atemluft-Schlauchabschnitt geführt sind. Bei den Mantel-Drähten kann es sich unter anderem um Heizdrähte des Atemluft-Schlauchabschnitts handeln. Die Mantel-Drähte können wendelförmig um ein Luftführungslumen des Atemluft-Schlauchabschnitts geführt sein. Alternativ oder zusätzlich zu Heizdrähten kann als derartiger Mantel-Draht ein Signaldraht zur Übermittlung eines Temperatursensorsignals ausgeführt sein. Es können über die Kontaktpins zum Beispiel zwei oder drei Mantel-Drähte elektrisch kontaktiert sein.

Eine Durchführung der Sensoraufnahme nach Anspruch 1 ermöglicht den Einsatz eines Temperatursensors, der die Beatmungslufttemperatur direkt im Luftführungslumen des Konnektors misst. Dies erlaubt eine schnelle Temperaturmessung.

Eine Sensor-Führungskomponente nach Anspruch 1 kann ein Einsetzen Temperatursensors in die Sensoraufnahme erleichtern. Die Sensor-Führungskomponente kann gleichzeitig einem Schutz des Temperatursensors dienen. Die Sensor-Führungskomponente kann mindestens ein Fenster aufweisen, damit ein Luftführungslumen der Luftführungs-Konnektorkomponente vom Temperatursensor her zur Temperaturmessung der im Luftführungslumen geführten Beatmungsluft zugänglich ist. Die Sensor-Führungskomponente kann thermisch-leitfähige Komponenten enthalten zur verbesserten Anbindung des Temperatursensors an das Luftführungslumen. Die Sensor-Führungskomponente kann integraler Bestandteil der Sensoraufnahme und/oder des Konnektorgehäuses sein.

Eine Sensoraufnahme nach Anspruch 1 ergibt eine sichere Halterung der Temperatursensoreinrichtung. Die Sensoraufnahme kann einen Rastabschnitt zum Verrasten mit einem entsprechenden Rastabschnitt einer den Temperatursensor aufweisenden Temperatursensoreinrichtung aufweisen.

Eine weitere Aufgabe der Erfindung ist es, eine Temperatursensoreinrichtung zum Einsatz mit der vorstehend beschriebenen Baugruppe zu schaffen.

Die Temperatursensoreinrichtung hat zunächst die Vorteile, die vorstehend im Zusammenhang mit der Baugruppe bereits erläutert wurden. Der Griffabschnitt ermöglicht ein sicheres Erfassen des Tragkörpers der Temperatursensoreinrichtung uns somit ein sicheres Einstecken des Temperatursensors in die Sensoraufnahme und/oder ein sicheres Entfernen der Temperatursensoreinrichtung, gegebenenfalls zum Austausch gegen einen Blind-Deckel. Kontaktpins der Temperatursensoreinrichtung, insbesondere buchsenartig, ermöglichen den elektrischen Kontakt zu den Kontaktpins der Sensoraufnahme. Der Tragkörper und der Griffabschnitt können einstückig miteinander verbunden sein und beispielsweise Ergebnis eines gemeinsamen Spritzgussprozesses sein.

Mit dem Temperatursensor ist insbesondere eine Temperaturwert-Schwellwerterkennung möglich. Dies kann zu medizinischen Zwecken, aber auch zu anderen Zwecken eingesetzt werden, wie beispielsweise zum Brandschutz. Eine Genauigkeit des Temperatursensors kann besser sein als 0,5 K und kann beispielsweise bei 0,2 K, bei 0,1 K oder auch bei 0,05 K liegen. Diese Genauigkeit kann in einem Bereich zwischen -10°C und 80°C gewährleistet sein. Auch ein engerer Temperaturbereich, in dem die Genauigkeit gewährleistet ist, ist möglich, beispielsweise zwischen 0°C und 50°C oder zwischen 10°C und 50°C.

Der Temperatursensor kann über den Tragkörper überstehen. Der Temperatursensor kann in den Tragkörper eingebettet sein.

Der Temperatursensor kann eine strömungsgünstige Grundform aufweisen, zum Beispiel eine Perlenform.

Eine Rastverbindung nach Anspruch 1 ermöglicht eine sichere mechanische Verbindung der Temperatursensoreinrichtung mit der Sensoraufnahme. Der Gegen-Rastabschnitt kann seinerseits an der Sensoraufnahme angeformt sein.

Ein Ausführungsbeispiel der Erfindung wird nachfolgend anhand der Zeichnung näher erläutert. In dieser zeigen:

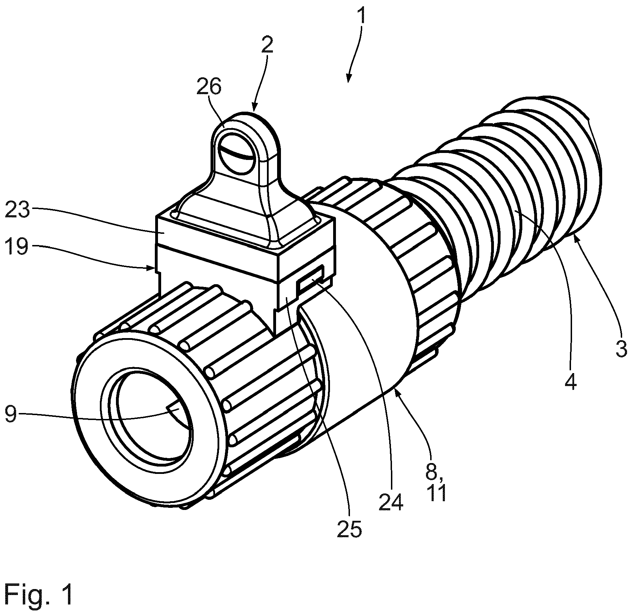

- Figur 1: perspektivisch eine Baugruppe für ein Patienten-Beatmungssystem mit einem Atemluft-Schlauchabschnitt, einem hiermit verbundenen Konnektor zum Verbinden des Atemluft-Schlauchabschnitts mit einem Patienten-Interface und einer Sensoraufnahme, in die eine Temperatursensoreinrichtung eingesetzt ist;

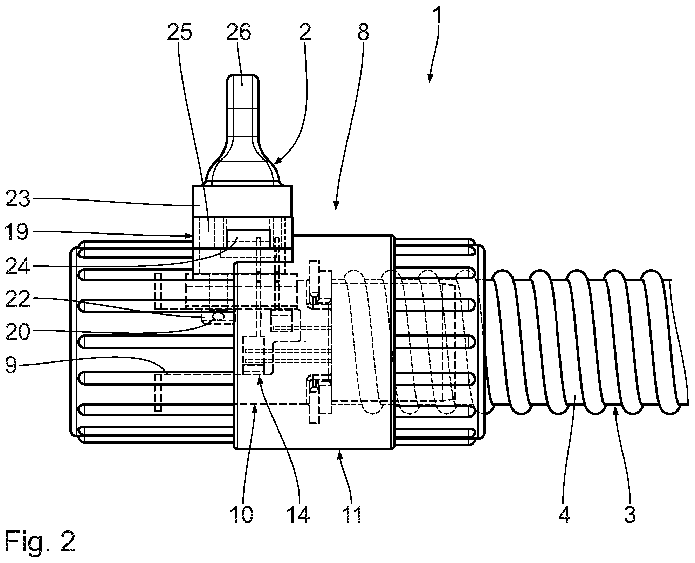

- Figur 2: eine innere Details preisgebende Seitenansicht der Baugruppe nach Figur 1, wiederum mit eingesteckter Temperatursensoreinrichtung;

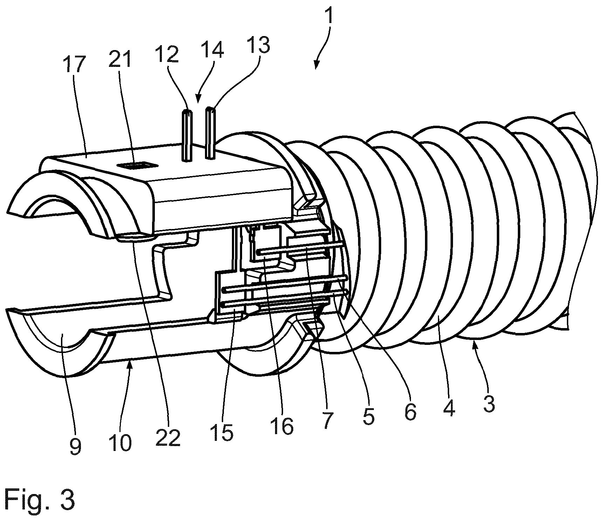

- Figur 3: wiederum perspektivisch die Baugruppe in einem Zwischenstadium ihrer Herstellung, wobei eine vorgeformte 3D-Leitungsverbindung jeweils einteilig mit Kontaktpins der Sensoraufnahme und Mantel-Drähten des Atemluft-Schlauchabschnitts zur Formung einer inneren Leitungsträger-Gehäusehülse des Konnektors umspritzt sind;

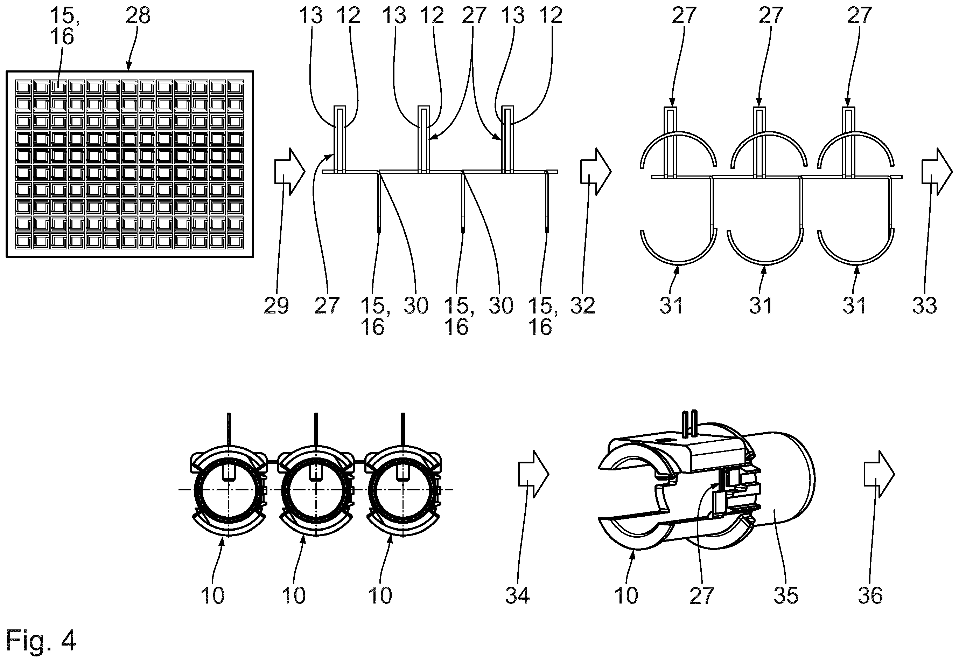

- Figur 4: teilweise schematisch Verarbeitungsschritte bei einem Verfahren zur Herstellung der Baugruppe einschließlich der Herstellung mehrerer innerer Leitungsträger-Gehäusehülsen durch Umspritzen und nachfolgend mechanische Trennung zunächst miteinander verbundener 3D-Leitungsverbindungen, die beim Umspritzschritt innerhalb einer Mehrfachkavität noch miteinander verbunden waren;

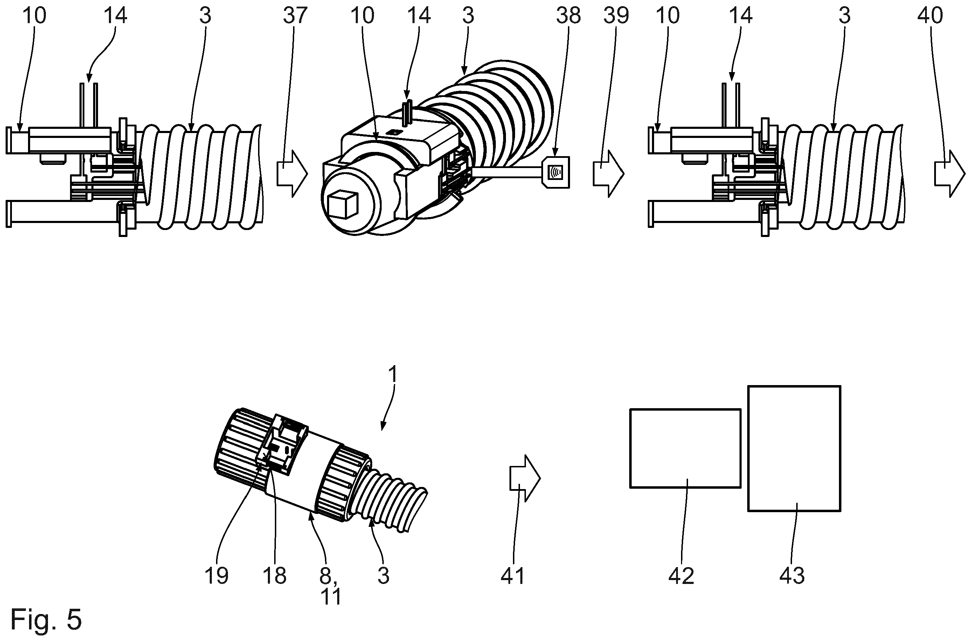

- Figur 5: weitere Verarbeitungsschritte des Herstellungsverfahrens bis zur Herstellung der fertigen Baugruppe und einer sich anschließenden Qualitätskontrolle;

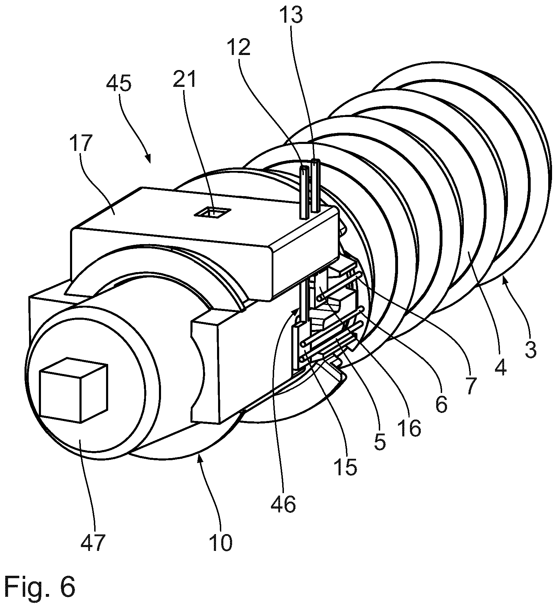

- Figur 6: in einer zu Figur 3 ähnlichen Darstellung, die zu dem einen Kern eines Ultraschallschweißwerkzeuges zeigt, eine weitere Ausführung der Baugruppe im Zwischenstadium ihrer Herstellung, wobei eine vorbereitete Leitungsverbindung, ausgeführt als 2D-Leitungsverbindung mit den Kontaktpins der Sensoraufnahme und den Mantel-Drähten des Atemluft-Schlauchabschnitts zur Formung der inneren Leitungsträger-Gehäusehülse des Konnektors umspritzt ist;

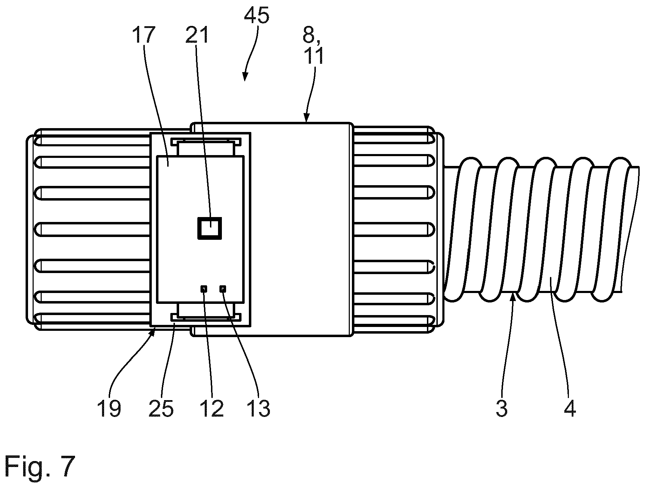

- Figur 7: eine Aufsicht auf die komplett hergestellte Baugruppe, ausgehend vom Zwischenstadium nach Figur 6, wobei die Baugruppe ohne eingesetzte Temperatursensoreinrichtung dargestellt ist.

Ein Patienten-Beatmungssystem, zu dem eine Baugruppe 1 gehört, die mit einer eingesteckten Temperatursensoreinrichtung 2 in der Figur 1 perspektivisch dargestellt ist, dient zur Beatmung eines Patienten im klinischen, sonstigen stationären oder auch häuslichen Pflegebereich. Grundsätzlich ist ein derartiges Patienten-Beatmungssystem bekannt aus der WO 2007/051 230 A1 und der DE 10 2019 216 489 A1. Vom Markt her ist ein entsprechendes Patienten-Beatmungssystem bekannt vom Produkt "VentStar Helix".

Die wesentlichen, luftführenden Komponenten des Beatmungssystems 1 sind aus Kunststoff.

Die Baugruppe 1 hat einen Atemluft-Schlauchabschnitt 3 zur Führung von Beatmungsluft von einer nicht dargestellten Atemluftquelle hin zu einem Patienten.

Längs eines Schlauchmantels 4 des Atemluft-Schlauchabschnitts 3 sind elektrisch leitfähige Mantel-Drähte 5, 6 und 7 (vgl. Figur 3) geführt. Diese Führung erfolgt wendelförmig um ein inneres Luftführungslumen des Atemluft-Schlauchabschnitts 3. Die Mantel-Drähte 5 bis 7 sind hierzu wendelförmig mit Material des Atemluft-Schlauchabschnitts umspritzt. Zwei dieser Mantel-Drähte, nämlich die Mantel-Drähte 5 und 6 stellen Heizdrähte für den Atemluft-Schlauchabschnitt 3 dar, die über eine nicht dargestellte Versorgungseinrichtung mit Heizstrom versorgt werden können. Der dritte Mantel-Draht 7 stellt eine Signalleitung für die Temperatursensoreinrichtung 2 dar.

Mit dem Atemluft-Schlauchabschnitt 3 ist ein Konnektor 8 der Baugruppe 1 durch Umspritzen des Atemluft-Schlauchabschnitts 3 verbunden. Der Konnektor 8 dient zum Verbinden des Atemluft-Schlauchabschnitts 3 mit einem in der Zeichnung nicht dargestellten Patienten-Interface des Patienten-Beatmungssystems. Diese Verbindung ist so, dass eine innere Luftführungs-Konnektorkomponente 9 des Konnektors 8 jedenfalls abschnittsweise einen integralen Bestandteil mit dem Atemluft-Schlauchabschnitt 3 bildet.

Der Konnektor 8 hat eine innere Leitungsträger-Gehäusehülse 10 und ein äußeres Konnektorgehäuse 11. Diejenigen Abschnitte der Luftführungs-Konnektorkomponente 9, die an den Atemluft-Schlauchabschnitt 3 angeformt und somit einen integralen Bestandteil mit diesem bilden, sind Abschnitte des äußeren Konnektorgehäuses 11 des Konnektors 8. Die Luftführungs-Konnektorkomponente 9 des Konnektors 8 und der Atemluft-Schlauchabschnitt 3 sind einstückig miteinander verbunden.

Eingeformt in die innere Leitungsträger-Gehäusehülse 10 sind Leitungskomponenten 12, 13 einer sich dreidimensional erstreckenden 3D-Leitungsverbindung der Baugruppe 1. Diese 3D-Leitungsverbindung wird nachfolgend noch erläutert.

Die Leitungskomponente 12 ist über ein Kontaktpad 15 mit den beiden Heiz-Mantel-Drähten 5, 6 elektrisch verbunden und stellt eine Kurzschlussbrücke für einen Heizkreis des Atemluft-Schlauchabschnitts 3 dar. Die Leitungskomponente 13 ist über ein Kontaktpad 16 elektrisch mit den Signal-Mantel-Draht 7 verbunden. Zum Kontaktieren mit den Kontaktpads 15, 16 sind die Mantel-Drähte 5 bis 7 aus der wendelförmigen Umspritzung freigelegt. Von den Kontaktpads 15, 16 abgewandt enden die Leitungskomponenten 12, 13 in Kontaktpins, die über eine isolierende Verbindungsplatte 17 der inneren Leitungsträger-Gehäusehülse 10 überstehen. Bei eingesteckter Temperatursensoreinrichtung 2 sind die Kontaktpins der Leitungskomponenten 12, 13 mit entsprechenden Kontaktbuchsen der Temperatursensoreinrichtung 2 elektrisch verbunden.

Die beiden überstehen Kontaktpins der Leitungskomponenten 12, 13 dienen zum elektrischen Konnektieren der Mantel-Drähte 5 bis 7 mit der Temperatursensoreinrichtung 2.

Zusammen mit einem Aufnahmekörper 18 (vgl. die vorletzte Abbildung der Figur 5), der Bestandteil des äußeren Konnektorgehäuses 11 ist, stellt die Verbindungsplatte 17 eine Sensoraufnahme 19 zum Einsetzen der Temperatursensoreinrichtung 2 in den Konnektor 8 und insbesondere zum Einsetzen eines Temperatursensors 20 der Temperatursensoreinrichtung 2 in die Luftführungs-Konnektorkomponente 9, also in dessen Luftführungslumen, dar. Die Sensoraufnahme 19 ist einstückig in das äußere Konnektorgehäuse 11 des Konnektors 8 eingeformt. Der Temperatursensor 20 dient zum Messen einer Temperatur der durch die Luftführungs-Konnektorkomponente 9 strömenden Beatmungsluft dar.

Die Temperatursensoreinrichtung 2 ist ein Beispiel für eine in die Aufnahme 19 einsteckbare Elektronikkomponente. Die Sensoraufnahme 19 ist daher allgemeiner eine Komponentenaufnahme. Anstelle eines Temperatursensors kann bei der Elektronikkomponente auch eine Sensoreinrichtung zur Erfassung eines weiteren Parameters, insbesondere eines Atemluft-Parameters, zum Einsatz kommen.

Bei der Sensoreinrichtung kann es sich einen Sensor zur Messung der Luftfeuchtigkeit oder auch um einen chemischen oder spektroskopischen Sensor handeln. Auch eine andere Elektronikkomponente kann in die Komponentenaufnahme 19 eingesetzt werden, beispielsweise eine Überwachungseinheit beispielsweise zur Bestimmung einer Gebrauchsdauer oder einer Gebrauchsbelastung der Baugruppe 1.

Die Sensoraufnahme 19 hat eine Durchführung 21 zum Durchführen des Temperatursensors 20 der Temperatursensoreinrichtung 2 in das Luftführungslumen der Luftführungs-Konnektorkomponente 9. Diese Durchführung 21 ist als Öffnung in der Verbindungsplatte 17 ausgeführt, die in das Luftführungslumen der Luftführungs-Konnektorkomponente 9 einmündet.

Zudem hat die Sensoraufnahme 19 eine Sensor-Führungskomponente 22, die unter anderem zur Führung einer Einsetzbewegung des Temperatursensors 20 in das Luftführungslumen der Luftführungs-Konnektorkomponente 9 dient. Die Sensor-Führungskomponente 22 stellt zudem einen Strömungs- und/oder Berührungsschutz für den Temperatursensor 20 dar.

Die Sensor-Führungskomponente 22 kann mindestens ein Fenster aufweisen, über das das Luftführungslumen vom Temperatursensor 20 her zur Temperaturmessung der im Luftführungslumen geführten Beatmungsluft zugänglich ist. Die Sensor-Führungskomponente 22 kann thermisch-leitfähige Komponenten enthalten zur thermischen Anbindung des Temperatursensors 20 an das Luftführungslumen.

Die Sensoraufnahme 19 ist zum steckbaren Einsetzen der Temperatursensoreinrichtung 2 ausgeführt. Hierzu hat die Temperatursensoreinrichtung 2 einen Tragkörper 23, über den der in die Sensoraufnahme 19 eingesteckte Temperatursensor 20 nach unten in das Luftführungslumen der Luftführungs-Konnektorkomponente 9 übersteht. An den Tragkörper 23 ist ein Rastabschnitt 24 (vgl. Figur 1) zum Verrasten mit einem Gegen-Rastabschnitt 25 der Sensoraufnahme 19 angeformt. Der Gegen-Rastabschnitt 25 wiederum ist angeformter Bestandteil der Sensoraufnahme 19.

Im Tragkörper 23 sind Kontaktpins der Temperatursensoreinrichtung zur Verbindung mit den Kontaktpins der Leitungskomponenten 12, 13 vorgesehen.

Ebenfalls an den Tragkörper 23 angeformt ist ein Griffabschnitt 26 der Temperatursensoreinrichtung 2 zum Erfassen eines vom Temperatursensor 20 abgewandten Abschnitts des Tragkörpers 23.

Ein Verfahren zum Herstellen der Baugruppe 1 wird nachfolgend anhand der Figuren 4 und 5 näher erläutert.

Zum Herstellen der Baugruppe 1 wird zunächst eine sich dreidimensional erstreckende Leitungsverbindung 27 ausgehend von einer als Leadframe ausgeführten 2D-Leiterstruktur 28 vorgeformt. Die Leitungskomponenten der 2D-Leitungsstruktur 28 können Querschnitte ausweisen, die kleiner sind als 0,5 mm und die im Bereich zwischen 50 µm und 200 µm liegen können.

Die jeweilige 3D-Leitungsverbindung 27 erstreckt sich zwischen den jeweiligen Kontaktpads 15, 16 und den Kontaktpins der Leitungskomponenten 12, 13. In der 2D-Leiterstruktur 28 sind eine Vielzahl von Kontaktpads 15 beziehungsweise 16 über entsprechende Leiterbahnen miteinander verbunden, die im Rahmen eines Vorformschritts 29 gegeneinander insbesondere um 90° zur 3D-Leitungsverbindung 27 aufgebogen werden.

Ergebnis des Vorformschritts 29 ist eine Mehrzahl von 3D-Leitungsverbindungen 27, im vorliegenden Beispiel 3 derartige 3D-Leitungsverbindungen 27, die einer entsprechenden Mehrzahl später anzuformender innerer Leitungsträger-Gehäusehülsen 10 eines jeweiligen Konnektors 8 zugeordnet sind. Diese mehreren 3D-Leitungsverbindungen 27 sind nach dem Vorformschritt 29 zunächst noch mechanisch über Sollbruchstellen 30 miteinander verbunden.

Nach dem Vorformen 29 der 3D-Leitungsverbindungen 27 werden die noch mechanisch miteinander verbundenen 3D-Leitungsverbindungen 27 in ein Spritzgusswerkzeug in Form einer Spritzguss-Mehrfachkavität 31 eingesetzt, was in einem Einsetzschritt 32 erfolgt. In Anschluss hieran geschieht in einem Umspritzschritt 33 ein Umspritzen der 3D-Leitungsverbindungen 27 zur Formung der inneren Leitungsträger-Gehäusehülsen 10 des jeweiligen Konnektors 8. Beim Umspritzschritt 33 wird gleichzeitig die noch mechanisch miteinander verbundene Mehrzahl der 3D-Leitungsverbindungen 27 zur Formung der entsprechenden Mehrzahl innerer Leitungsträger-Gehäusehülsen 10 in der Spritzguss-Mehrfachkavität 31 umspritzt.

Nach dem Umspritzschritt 33 erfolgt in einem Trennschritt 34 eine mechanische Trennung der miteinander verbundenen 3D-Leitungsverbindungen 27 an den Sollbruchstellen 30 und damit eine Vereinzelung der inneren Leitungsträger-Gehäusehülsen 10.

Im Anschluss hieran wird eine Anschlusshülse 35 der vereinzelten inneren Leitungsträger-Gehäusehülse 10 in einen zugewandten Endabschnitt des Atemluft-Schlauchabschnitts 3 in einem Einsteckschritt 36 eingesteckt.

Figur 5 zeigt links oben eine entsprechend in den Atemluft-Schlauchabschnitt 3 eingesteckte innere Leitungsträger-Gehäusehülse 10. Im Rahmen des Einsteckschritts 36 werden die Kontaktpads 15, 16 der Leitungskomponenten 12, 13 zu den freigelegten Endabschnitten der Mantel-Drähte 5 bis 7 hin positioniert und ausgerichtet.

In einem nachfolgenden Verbindungsschritt 37 werden die Leitungskomponenten 12, 13 der Leitungsverbindung 14 beziehungsweise 27 mit den Endabschnitten der Mantel-Drähte 5 bis 7 elektrisch kontaktiert. Dies erfolgt mittels Ultraschall-Schweißen mit einer entsprechenden Ultraschall-Schweißeinrichtung 38.

Anschließend wird in einem Einlegeschritt 39 die vorgefertigte Roh-Baugruppe mit der inneren Leitungsträger-Gehäusehülse 10 und dem hiermit mechanisch und elektrisch verbundenen Atemluft-Schlauchabschnitt 3 in ein weiteres Spritzgusswerkzeug eingelegt. In einem anschließenden weiteren Umspritzschritt 40 wird dann die innere Leitungsträger-Gehäusehülse 10 sowie ein hieran angrenzender Endbereich des Atemluft-Schlauchabschnitts 3 zur Formung des äußeren Konnektorgehäuses 11 umspritzt. Hierbei wird auch die Sensoraufnahme 19 im äußeren Konnektorgehäuse geformt.

Bei der Elektronikkomponente, die in der Komponenten- beziehungsweise Sensoraufnahme 19 aufgenommen werden kann, kann es sich allgemein um eine Sensoreinrichtung handeln, also beispielsweise um die Temperatursensoreinrichtung 2.

Nach dem weiteren Umspritzschritt 40 erfolgt in einem Kontrollschritt 41 eine optische Qualitätskontrolle 42 und eine elektrische Qualitätskontrolle 43, wobei die Baugruppe 1 einer Sichtprüfung sowie einer Überprüfung durch entsprechende optische und/oder elektrische/elektronische Messeinheiten unterzogen werden kann.

Anhand der Figuren 6 und 7 wird nachfolgend eine weitere Ausführung 45 der Baugruppe für das Patienten-Beatmungssystem beschrieben, die anstelle der Baugruppe zum Einsatz kommen kann, die vorstehend anhand der Figuren 1 bis 5 beschrieben wurde. Komponenten und Funktionen, die denjenigen entsprechen, die vorstehend in Bezugnahme auf die Figuren 1 bis 5 bereits erläutert wurden, tragen die gleichen Bezugsziffern und werden nicht nochmals im Einzelnen diskutiert.

Bei der Baugruppe 45 ist eine Leitungsverbindung 46, die ansonsten der 3D-Leitungsverbindung 27 der Ausführung nach den Figuren 1 bis 5 entspricht, zweidimensional ausgeführt. Die Kontaktpins 12, 13 dieser Leitungsverbindung sowie die Kontaktpads 15,16 liegen in einer gemeinsamen Anordnungsebene der 2D-Leitungsverbindung 46.

Figur 6 zeigt zudem einen Kern 47 eines Ultraschallschweißwerkzeuges zum Ultraschallschweißen der Leitungskomponenten 12, 13 der Leitungsverbindung 14 beziehungsweise 27 mit den Endabschnitten der Mantel-Drähte 5 bis 7 der Baugruppe 45.

Das Herstellungsverfahren der Baugruppe 45 entspricht grundsätzlich demjenigen, das vorstehend anhand der Figuren 4 und 5 bereits erläutert wurde.

Anstelle einer Vorformung einer 3D-Leitungsverbindung erfolgt beim Herstellen der Baugruppe 45 zunächst eine Bereitstellung der 2D-Leitungsverbindung 46 ausgehend von der als Leadframe ausgeführten 2D-Leiterstruktur 28. Dieses Bereitstellen kann ausschließlich durch Trennen der zugehörigen Leitungskomponenten von umgebenden Trag- beziehungsweise Leitungskomponenten des Leadframes 28 geschehen.

Ergebnis dieser Bereitstellung der 2D-Leitungsverbindungen 46 ist eine Mehrzahl derartiger 2D-Leitungsverbindungen 46, zum Beispiel drei derartige 2D-Leitungsverbindungen 46, die einer entsprechenden Mehrzahl der später anzuformenden inneren Leitungsträger-Gehäusehülsen 10 des jeweiligen Konnektors 8 der Baugruppe 45 zugeordnet sind. Diese mehreren 2D-Leitungsverbindungen 46 sind nach dem Bereitstellungsschritt zunächst noch mechanisch über Sollbruchstellen miteinander verbunden, wie vorstehend anhand der 3D-Leitungsverbindungen 27 der Ausführung nach den Figuren 1 bis 4 bereits erläutert.

Es erfolgt dann bei der Herstellung der Baugruppe 45 das Einsetzen 32, das Umspritzen, das Trennen 34, das Einstecken der Anschlusshülse 35 einschließlich des Positionierens und Ausrichtens der Kontaktpads 15, 16 zu den beigelegten Endabschnitten der Mantel-Drähte 5 bis 7, das elektrische Verbinden 37, das Einlegen 39, das weitere Umspritzen 40 sowie die Kontrolle 41 entsprechend dem, was vorstehend im Zusammenhang mit der Herstellung der Baugruppe 1 nach den Figuren 1 bis 5 bereits erläutert wurde.

## Patentansprüche

1. Baugruppe (1; 45) für ein Patienten-Beatmungssystem
- mit einem Atemluft-Schlauchabschnitt (3) zur Führung von Beatmungsluft von einer Beatmungsquelle hin zu einem Patienten,
- mit einem mit dem Atemluft-Schlauchabschnitt (3) verbundenen Konnektor (8) zum Verbinden des Atemluft-Schlauchabschnitts (3) mit einer weiteren, die Beatmungsluft führenden Komponente des Patienten-Beatmungssystems, wobei eine Luftführungs-Konnektorkomponente (9) des Konnektors (8) einen integralen Bestandteil mit dem Atemluft-Schlauchabschnitt (3) bildet, und
- mit einer Temperatursensoreinrichtung (2) zum Messen einer Temperatur der Beatmungsluft;
- wobei der Konnektor (8) eine Sensoraufnahme (19) zum steckbaren Einsetzen der Temperatursensoreinrichtung (2) in die Luftführungs-Konnektorkomponente (9) aufweist;
- wobei die Sensoraufnahme (19) eine Sensor-Führungskomponente (22) zur Führung einer Einsetzbewegung der Temperatursensoreinrichtung (2) in das Luftführungslumen der Luftführungs-Konnektorkomponente (9) aufweist,
- wobei die Temperatursensoreinrichtung (2) einen Tragkörper (23) aufweist; und
- wobei längs eines Schlauchmantels (4) des Atemluft-Schlauchabschnitts (3) elektrisch leitfähige Mantel-Drähte (5, 6, 7) geführt sind, von denen zwei Mantel-Drähte (5, 6) Heizdrähte für den Atemluft-Schlauchabschnitt (3) darstellen;
**dadurch gekennzeichnet, dass**
- ein dritter Mantel-Draht (7) der elektrisch leitfähige Mantel-Drähte (5, 6, 7) eine Signalleitung für die Temperatursensoreinrichtung (2) darstellt;
- die Sensoraufnahme (19) eine Durchführung (21) zum Durchführen der Temperatursensoreinrichtung (2) in ein Luftführungslumen der Luftführungs-Konnektorkomponente (9) aufweist,
- die Sensor-Führungskomponente (22) mindestens ein Fenster aufweist, über das das Luftführungslumen von der Temperatursensoreinrichtung (2) her zur Temperaturmessung der im Luftführungslumen geführten Beatmungsluft zugänglich ist;
- an den Tragkörper (23) der Temperatursensoreinrichtung (2) ein Rastabschnitt (24) angeformt ist, zum Verrasten mit einem Gegen-Rastabschnitt (25) der Sensoraufnahme (19);
- der Gegen-Rastabschnitt (25) angeformter Bestandteil der Sensoraufnahme (19) ist
- der Konnektor (8) eine innere Leitungsträger-Gehäusehülse (10) und ein äußeres Konnektorgehäuse (11) aufweist;
- in die innere Leitungsträger-Gehäusehülse (10) Leitungskomponenten (12, 13) eingeformt sind;
- eine erste Leitungskomponente (12) über ein Kontaktpad (15) mit den beiden Heiz-Mantel-Drähten (5, 6) elektrisch verbunden ist;
- eine zweite Leitungskomponente (13) über ein Kontaktpad (16) elektrisch mit dem Signal-Mantel-Draht (7) verbunden ist; und
- die erste und die zweite Leitungskomponente (12, 13) überstehende Kontaktpins aufweisen, die bei eingesteckter Temperatursensoreinrichtung (2) mit entsprechenden Kontaktbuchsen der Temperatursensoreinrichtung (2) elektrisch verbunden sind.

2. Baugruppe nach einem der Ansprüche 1 bis 3, **dadurch gekennzeichnet, dass** ein Temperatursensor (20) Bestandteil der Temperatursensoreinrichtung (2) ist.

3. Baugruppe (1) nach Anspruch 1 oder 2, weiterhin aufweisend einen Griffabschnitt (26) zum Erfassen eines vom Temperatursensor (20) abgewandten Abschnitts des Tragkörpers (23).

## Claims

1. An assembly (1; 45) for a patient ventilation system
- having a respiratory air hose section (3) for guiding ventilation air from a ventilation source to a patient,
- having a connector (8), connected to the respiratory air hose section (3), for connecting the respiratory air hose section (3) to a further component of the patient ventilation system guiding the ventilation air, wherein an air guiding connector component (9) of the connector (8) forms an integral component with the respiratory air hose section (3), and
- having a temperature sensor device (2) for measuring a temperature of the ventilation air;
- wherein the connector (8) has a sensor receptacle (19) for pluggable insertion of the temperature sensor device (2) into the air guiding connector component (9);
- wherein the sensor receptacle (19) has a sensor guiding component (22) for guiding an insertion movement of the temperature sensor (20) into the air guiding lumen of the air guiding connector component (9);
- wherein the temperature sensor device (2) comprises a support body (23); and
- wherein electrically conductive jacketed wires (5, 6, 7) are guided along a hose jacket (4) of the respiratory air hose section (3), two of which represent heating wires (5, 6) for the respiratory air hose section (3);
**characterized in that**
- a third jacketed wire (7) of the electrically conductive jacketed wires (5, 6, 7) represents a signal line for the temperature sensor device (2);
- the sensor receptacle (19) has a feedthrough (21) for feeding through the temperature sensor device (2) into an air guiding lumen of the air guiding connector component (9);
- the sensor guiding component (22) has at least one window, via which the air guiding lumen is accessible from the temperature sensor device (2) for temperature measurement of the ventilation air guided in the air guiding lumen;
- a detent section (24) for locking with a counter detent section (25) of the sensor receptacle (19) is formed on the support body (23)of the temperature sensor device (2);
- the counter detent section (25) is a component formed on the sensor receptacle (19);
- the connector (8) comprises an inner line carrier housing sleeve (10) and an outer connector housing (11);
- line components (12, 13) are moulded into the inner line carrier housing sleeve (10);
- a first line component (12) is electrically connected via a contact pad (15) to the two heating jacketed wires (5, 6);
- a second line component (13) is electrically connected via a contact pad (16) to the signal jacketed wire (7);
- the first and second line components (12, 13) comprise protruding contact pins, which are electrically connected to corresponding contact sockets of the temperature sensor device (2), when the temperature sensor device (2) is plugged in.

2. The assembly according to Claim 1, wherein a temperature sensor (20) is part of the temperature sensor device (2).

3. The assembly according to Claim 1 or 2, further comprising a handle section (26) for grasping a section of the support body (23) facing away from the temperature sensor (20).

## Revendications

1. Ensemble (1 ; 45) pour un système respiratoire de patient
- avec une section de tuyau d'air respirable (3) pour acheminer de l'air de ventilation d'une source d'air respirable à un patient,
- avec un connecteur (8) relié à la section de tuyau d'air respirable (3) pour raccorder la section de tuyau d'air respirable (3) avec un autre composant conduisant l'air de ventilation du système respiratoire de patient, dans lequel un composant de conduite d'air (9) du connecteur (8) fait partie intégrante de la section de tuyau d'air respirable (3), et
- avec un dispositif de capteur de température (2) pour mesurer la température de l'air de ventilation ;
- dans lequel le connecteur (8) présente un logement de capteur (19) pour l'insertion enfichable du dispositif de capteur de température (2) dans le composant de conduite d'air (9) du connecteur ;
- dans lequel le logement de capteur (19) présente un composant de guidage de capteur (22) destiné à guider un mouvement d'insertion du dispositif de capteur de température (2) dans la lumière de guidage d'air du composant de guidage d'air (9) du connecteur,
- dans lequel le dispositif de capteur de température (2) présente un corps de support (23) ; et
- dans lequel des fils de gaine électriquement conducteurs (5, 6, 7) sont guidés le long d'une gaine (4) de la section de tuyau d'air respirable (3), dont deux fils de gaine (5, 6) constituent des fils chauffants pour la section de tuyau d'air respirable (3) ;
**caractérisé en ce que**
- un troisième fil de gaine (7) des fils de gaine électriquement conducteurs (5, 6, 7) représente une ligne de signalisation pour le dispositif de capteur de température (2) ;
- le logement de capteur (19) présente un passage (21) pour faire passer le dispositif de capteur de température (2) dans une lumière de guidage d'air du composant de guidage d'air (9) du connecteur,
- le composant de guidage de capteur (22) présente au moins une fenêtre à travers laquelle la lumière de guidage d'air est accessible depuis le dispositif de capteur de température (2) pour mesurer la température de l'air de ventilation guidé dans la lumière de guidage d'air ;
- une section d'encliquetage (24) est formée sur le corps de support (23) du dispositif de capteur de température (2) afin de s'encliqueter avec une section d'encliquetage complémentaire (25) du logement de capteur (19) ;
- la section d'encliquetage complémentaire (25) est un élément moulé du logement du capteur (19) ;
- le connecteur (8) présente une douille de boîtier de porte-conducteur interne (10) et un boîtier externe (11) du connecteur ;
- des composants de conduite (12, 13) sont moulés dans la douille de boîtier de porte-conducteur interne (10) ;
- un premier composant de ligne (12) est relié électriquement aux deux fils de gaine chauffants (5, 6) via un plot de contact (15) ;
- un deuxième composant de ligne (13) est relié électriquement au fil de gaine de signalisation (7) via un plot de contact (16) ; et
- le premier et le deuxième composant de ligne (12, 13) présentent des broches de contact saillantes qui, lorsque le dispositif de capteur de température (2) est enfiché, sont reliées électriquement à des douilles de contact correspondantes du dispositif de capteur de température (2).

2. L'ensemble selon l'une quelconque des revendications 1 à 3, **caractérisé en ce qu'**un capteur de température (20) fait partie intégrante du dispositif de capteur de température (2).

3. L'ensemble (1) selon la revendication 1 ou 2, présentant en outre une section de préhension (26) pour saisir une partie du corps de support (23) opposée au capteur de température (20).
